# EUROPÄISCHE PATENTSCHRIFT

(11) **EP 2 783 676 B1**
(45) Veröffentlichungstag und Bekanntmachung des Hinweises auf die Patenterteilung: **22.03.2017**
(21) Anmeldenummer: 14150914.1
(22) Anmeldetag: 13.01.2014
(51) Int. Cl.: A61K 8/34, A61K 8/37, A61Q 19/00, A61K 8/97, A61Q 19/08

(54) **Farbstabile kosmetische Zubereitung**
Colour-stable cosmetic preparation
Préparation cosmétique à couleur stabilisée

(30) Priorität: 28.03.2013 DE 102013205603
(43) Veröffentlichungstag der Anmeldung: 01.10.2014
(73) Patentinhaber: Beiersdorf AG, 20253 Hamburg (DE)
(72) Erfinder: Steikert Claudia, 20255 Hamburg (DE); Alanya-Rousseau, Esma, D-22769 Hamburg (DE); Detert, Marion, 22455 Hamburg (DE)

(56) Entgegenhaltungen:
- WO-A1-2012/116391
- WO-A2-2011/106493
- DE-A1-102004 003 437
- DE-A1-102010 015 790
- US-A1- 2004 081 713

## Beschreibung

Die vorliegende Erfindung betrifft eine kosmetische Zubereitung enthaltend Magnolia Officinalis Bark Extract und ein oder mehrere Öle mit einer Polarität von 20 mN/m bis 30mN/m.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren immer das Ziel der Menschen gewesen. Einen wesentlichen Anteil an einem schönen und attraktiven Äußeren hat dabei der Zustand und das Aussehen der Haut.

Damit die Haut ihre biologischen Funktionen im vollen Umfang erfüllen kann, bedarf sie der regelmäßigen Reinigung und Pflege. Die Reinigung der Haut dient dabei der Entfernung von Schmutz, Schweiß und Resten abgestorbener Hautpartikel, die einen idealen Nährboden für Krankheitserreger und Parasiten aller Art bilden. Hautpflegeprodukte dienen meist der Befeuchtung und Rückfettung der Haut. Häufig sind ihnen Wirkstoffe zugesetzt, welche die Haut regenerieren und beispielsweise ihre vorzeitige Alterung (z.B. das Entstehen von Fältchen und Falten) verhindern und vermindern sollen. Einer dieser Wirkstoffe ist der Magnolia Officinalis Bark Extract. Dieser Extrakt wird mit Hilfe der CO₂-Extraktion aus den Rinden der *Magnolia officinalis* gewonnen. Er enthält die Wirkstoffe

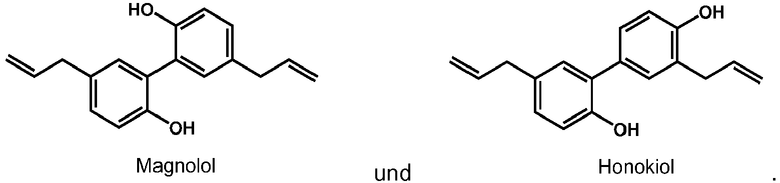

Nachteilig am Stande der Technik ist jedoch der Umstand, dass sich dieser Wirkstoff nicht temperaturstabil in kosmetische Zubereitungen, insbesondere O/W-Emulsionen einarbeiten lässt. Bei höheren Lagertemperaturen, wie sie beispielsweise im Sommer anzutreffen sind, kommt es zu einer Rosaverfärbung der ansonsten weißen Emulsion.

Ohne an diese Erklärung gebunden zu sein, deuten nähere Untersuchungen dieses Phänomens mit Hilfe der Raman Imaging Methode darauf hin, dass dieser Effekt wohl darauf zurück zu führen sein könnte, dass der Extrakt bzw. seine wesentlichen Inhaltsstoffe Magnolol und Honokiol bei höheren Temperaturen aus der Zubereitung ausfallen statt in Lösung zu bleiben.

Es war daher die Aufgabe der vorliegenden Erfindung, mit Hilfe der CO₂-Extraktion aus den Rinden der *Magnolia officinalis* gewonnen Magnolia Officinalis Bark Extract und insbesondere seine wesentlichen Inhaltsstoffe Magnolol und Honokiol temperaturstabil in kosmetische Zubereitungen, insbesondere O/W-Emulsionen einzuarbeiten. Insbesondere war es die Aufgabe der vorliegenden Erfindung, die lager- bzw. wärmeinduzierte Verfärbung derartiger Zubereitungen zu verhindern.

Überraschend gelöst wird die Aufgabe durch eine kosmetische Zubereitung enthaltend
a) Magnolia Officinalis Bark Extract und
b) ein oder mehrere Öle mit einer Polarität von 20 mN/m bis 30mN/m,
dadurch gekennzeichnet, dass die Öle mit einer Polarität von 20 mN/m bis 30mN/m gewählt werden aus der Gruppe der Verbindungen der Ethylhexylester von Fettsäuren.

Überraschend gelöst wird die Aufgabe durch ferner durch eine kosmetische Zubereitung enthaltend
a) 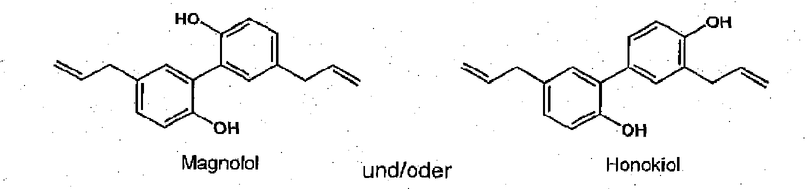 sowie
b) ein oder mehrere Öle mit einer Polarität von 20 mN/m bis 30mN/m,
dadurch gekennzeichnet, dass die Öle mit einer Polarität von 20 mN/m bis 30mN/m gewählt werden aus der Gruppe der Verbindungen der Ethylhexylester von Fettsäuren.

Erfindungsgemäß ist auch das Verfahren zur Temperatur- bzw. Farbstabilisierung von Magnolia Officinalis Bark Extract beziehungsweise von Magnolol und Honokiol in kosmetischen Zubereitungen, insbesondere O/W-Emulsionen mit Hilfe von Ölen mit einer Polarität von 20 mN/m bis 30mN/m, wie sie im Rahmen der vorliegenden Offenbarung näher charakterisiert werden.

Es ist erfindungsgemäß besonders vorteilhaft, wenn die Zubereitung Magnoila Offizinalis Bark Extract in einer Konzentration von 0,01 bis 0,5 Gewichts-%, bezogen auf das Gesamtgewicht der Zubereitung, enthält.

Unter der Polarität der erfindungsgemäßen Öle ist dabei die Grenzflächenspannung gegenüber Wasser zu verstehen. Sie kann beispielsweise mit dem Krüss K 10 Ringtensiometer bestimmt werden oder Tabellenwerken, wie sie beispielsweise in der DE 102004003437.0 offenbart sind, entnommen werden.

Zwar kennt der Stand der Technik die DE 102010015790, US 2004/081713, WO 2012/116391, WO 2011/106493 und DE 102004003437, doch konnten diese Schriften nicht den Weg zur vorliegenden Erfindung weisen.

Erfindungsgemäß besonders bevorzugte Ausführungsformen der vorliegenden Erfindung sind dadurch gekennzeichnet, dass die Öle mit einer Polarität von 20 mN/m bis 30mN/m gewählt werden aus der Gruppe der Verbindungen Ethylhexylcocoat (INCI Ethylhexyl Cocoate) und Ethylhexylstearat (INCI Ethylhexyl Stearate).

Es ist vorteilhaft im Sinne der vorliegenden Erfindung, wenn die Zubereitung Öle mit einer Polarität von bis in einer Gesamtkonzentration von 0,5 bis 5 Gewichts-%, bezogen auf das Gesamtgewicht der Zubereitung, enthält.

Die erfindungsgemäß besonders bevorzugten Einsatzkonzentrationen für Ethylhexylcocoat (INCI Ethylhexyl Cocoate) und Ethylhexylstearat (INCI Ethylhexyl Stearate) betragen jeweils (d.h. für jeden Stoff einzeln betrachtet) 0,5 bis 2 Gewichts-%, bezogen auf das Gesamtgewicht der Zubereitung.

Es ist erfindungsgemäß vorteilhaft, wenn die erfindungsgemäße Zubereitung eine Emulsion darstellt und erfindungsgemäß besonders bevorzugt, wenn die Zubereitung in Form einer O/W-Emulsion vorliegt.

Erfindungsgemäß vorteilhafte Ausführungsformen der vorliegenden Erfindung sind dadurch gekennzeichnet, dass die Zubereitung Natriumstearoylglutamat enthält.

In einem solchen Falle ist es erfindungsgemäß von Vorteil, wenn die Zubereitung Natriumstearoylglutamat in einer Konzentration von 0,1 bis 0,3 Gewichts-%, bezogen auf das Gesamtgewicht der Zubereitung, enthält.

Ferner ist es erfindungsgemäß vorteilhaft, wenn die erfindungsgemäße Zubereitung Cyclomethicon enthält.

In einem solchen Falle ist es erfindungsgemäß von Vorteil, wenn die Zubereitung Cyclomethicon in einer Konzentration von 4 bis 18 Gewichts-%, bezogen auf das Gesamtgewicht der Zubereitung, enthält.

Neben diesen Inhaltsstoffen kann die erfindungsgemäße Zubereitung noch weitere lipophile Komponenten enthalten.

Die Wasserphase der erfindungsgemäßen Zubereitungen kann -neben Wasser- vorteilhaft übliche kosmetische Hilfsstoffe enthalten, wie beispielsweise Alkohole, insbesondere solche niedriger C-Zahl wie Ethanol, Isopropanol, Diole oder Polyole niedriger C-Zahl sowie deren Ether, vorzugsweise Propylenglykol, Butylenglykol, 2-Methylpropan-1,3-diol, Pentan-1,2-diol, Hexan-1,2-diol, Octan-1,2-diol, Decan-1,2-diol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Elektrolyte, etc.. Die erfindungsgemäße Zubereitung kann vorteilhaft Konsistenzbildner (Gelbildner, Verdickungsmittel) wie beispielsweise Polyacrylate (auch quervernetzt) oder Cellulosederivate (beispielsweise Hydroxyethylcellulose) oder andere enthalten.

Erfindungsgemäß bevorzugt ist es, wenn die erfindungsgemäße Zubereitung dadurch gekennzeichnet ist, dass die Zubereitung Propylenglycol, Butylenglycol, 2-Methylpropan-1,3-diol, 1,2-Pentandiol, 1,2-Hexandiol, 1,2-Octandiol und/oder 1,2-Decandiol enthält. Dabei werden diese Diole/Glycole erfindungsgemäß vorteilhaft in einer Gesamtmenge von 0,25 bis 4 Gewichts-%, bezogen auf das Gesamtgewicht der Zubereitung, eingesetzt.

Selbstverständlich kann die Zubereitung mit den üblichen, in der Kosmetik verwendeten Konservierungsmitteln konserviert werden.

Dabei ist es erfindungsgemäß bevorzugt, wenn die Zubereitung Phenoxyethanol enthält.

Darüber hinaus ist es erfindungsgemäß bevorzugt, wenn die Zubereitung frei ist von Parabenen.

Erfindungsgemäß bevorzugt ist die erfindungsgemäße Zubereitung frei ist von Polyethylenglycolen.

Es ist erfindungsgemäß vorteilhaft, wenn die erfindungsgemäße Zubereitung einen oder mehrere Wirkstoffe gewählt aus der Gruppe der Verbindungen Gylcyrrhetinsäure, Harnstoff, Arctiin, alpha-Liponsäure, Folsäure, Phytoen, D-Biotin, alpha-Glucosylrutin, Carnitin, Carnosin, Coffein, natürliche und/oder synthetische Isoflavonoide, Glycerylglucose, Kreatin, Kreatinin, Taurin, Tocopherol, Tocopherolacetat, ß-Alanin und/oder Licochalcon A, enthält.

Darüber hinaus kann die erfindungsgemäße Zubereitung einen oder mehrere UV-Filter enthalten, beispielsweise 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)ben-zolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 4-(tert.-Butyl)-4'-methoxydibenzoylmethan; 2-Ethylhexyl-2-cyano-3,3-diphenylacrylat; 4-(Dimethylamino)-benzoesäure(2-ethylhexyl)ester; 4-(Dimethylamino)benzoesäure-amylester; 4-Methoxybenzalmalon-säuredi(2-ethylhexyl)-ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; Dioctylbutylamidotriazon (INCI: Diethylhexyl-Butamidotriazone); 2,4-bis-[5-1(dimethyl-propyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4-Bis-{[4-(2-ethyl-hexyloxy)-2-hydroxy]-phenyl}-6-(4-methoxyphenyl)-1,3,5-triazin; 2,4,6-Tribiphenyl-4-yl-1,3,5-triazin; Merocyanine; Titandioxid; Zinkoxid.

Die erfindungsgemäße Zubereitung kann insbesondere vorteilhaft als Tages- oder Nachtcreme verwendet werden.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **inci** | **1** | **2** | **3** |
|---|---|---|---|
| Creatine | 0,1 | 0,1 | 0,1 |
| 1-Methylhydantoin-2-Imide | 0,01 | 0,01 | 0,01 |
| Magnolia Officinalis Bark Extract | 0,05 | 0,01 | 0,5 |
| Sodium Hyaluronate | 0,15 | 0,15 | 0,15 |
| Cetearyl Alcohol | 1 | 1 | 1 |
| Cyclomethicone | 10 | 13 | 4 |
| Ethylhexyl Stearate | 3 | 2 | 2 |
| Cyclomethicone + Dimethiconol | 5 | 5 | 5 |
| Ethylhexyl Cocoate | 2 | 2 | 3 |
| Sodium Stearoyl Glutamate | 0,3 | 0,1 | 0,2 |
| Polymethylsilsesquioxane | 4 | 4 | 4 |
| Parfum | 0,4 | 0,4 | 0,4 |
| Glycerin | 9 | 9 | 9 |
| Aqua + Sodium Hydroxide | 0,58 | 0,58 | 0,58 |
| Phenoxyethanol | 0,6 | 0,6 | 0,6 |
| Methylpropanediol | 2 | 2 | 2 |
| 1,2-Hexanediol | 0,7 | 0,7 | 0,7 |
| Acrylates/C10-30 Alkyl Acrylate Crosspolymer | 0,34 | 0,34 | 0,34 |
| Acrylates/C10-30 Alkyl Acrylate Crosspolymer | 0,23 | 0,23 | 0,23 |
| Aqua | 60,54 | 58,78 | 66,19 |

## Patentansprüche

1. Kosmetische Zubereitung enthaltend
a) Magnolia Officinalis Bark Extract und
b) ein oder mehrere Öle mit einer Polarität von 20 mN/m bis 30mN/m, **dadurch gekennzeichnet, dass** die Öle mit einer Polarität von 20 mN/m bis 30mN/m gewählt werden aus der Gruppe der Verbindungen der Ethylhexylester von Fettsäuren.

2. Kosmetische Zubereitung enthaltend
a) 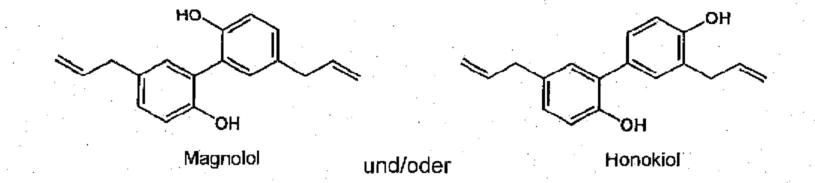 sowie
b) ein oder mehrere Öle mit einer Polarität von 20 mN/m bis 30mN/m, **dadurch gekennzeichnet, dass** die Öle mit einer Polarität von 20 mN/m bis 30mN/m gewählt werden aus der Gruppe der Verbindungen der Ethylhexylester von Fettsäuren.

3. Kosmetische Zubereitung nach einem der vorhergehenden Ansprüche, **dadurch gekennzeichnet, dass** die Öle mit einer Polarität von 20 mN/m bis 30mN/m gewählt werden aus der Gruppe der Verbindungen Ethylhexylcocoat (INCI Ethylhexyl Cocoate) und Ethylhexylstearat (INCI Ethylhexyl Stearate).

4. Kosmetische Zubereitung nach einem der vorhergehenden Ansprüche, **dadurch gekennzeichnet, dass** die Zubereitung Öle mit einer Polarität von bis in einer Gesamtkonzentration von 0,5 bis 5 Gewichts-%, bezogen auf das Gesamtgewicht der Zubereitung, enthält.

5. Kosmetische Zubereitung nach einem der vorhergehenden Ansprüche, **dadurch gekennzeichnet, dass** die Zubereitung in Form einer O/W-Emulsion vorliegt.

6. Kosmetisch Zubereitung nach einem der vorhergehenden Ansprüche, **dadurch gekennzeichnet, dass** die Zubereitung Natriumstearoylglutamat enthält.

7. Kosmetisch Zubereitung nach einem der vorhergehenden Ansprüche, **dadurch gekennzeichnet, dass** die Zubereitung Natriumstearoylglutamat in einer Konzentration von 0,1 bis 0,3 Gewichts-%, bezogen auf das Gesamtgewicht der Zubereitung, enthält.

8. Kosmetisch Zubereitung nach einem der vorhergehenden Ansprüche, **dadurch gekennzeichnet, dass** die Zubereitung Cyclomethicone enthält.

9. Kosmetisch Zubereitung nach einem der vorhergehenden Ansprüche, **dadurch gekennzeichnet, dass** die Zubereitung Cyclomethicon in einer Konzentration von 4 bis 18 Gewichts-%, bezogen auf das Gesamtgewicht der Zubereitung, enthält.

10. Kosmetisch Zubereitung nach einem der vorhergehenden Ansprüche, **dadurch gekennzeichnet, dass** die Zubereitung Magnoila Offizinalis Bark Extract in einer Konzentration von 0,01 bis 0,5 Gewichts-%, bezogen auf das Gesamtgewicht der Zubereitung, enthält.

## Claims

1. Cosmetic preparation comprising
a) magnolia officinalis bark extract and
b) one or more oils having a polarity of 20 mN/m to 30 mN/m, **characterized in that** the oils having a polarity of 20 mN/m to 30 mN/m are selected from the group of compounds of ethylhexyl esters of fatty acids.

2. Cosmetic preparation comprising
a) 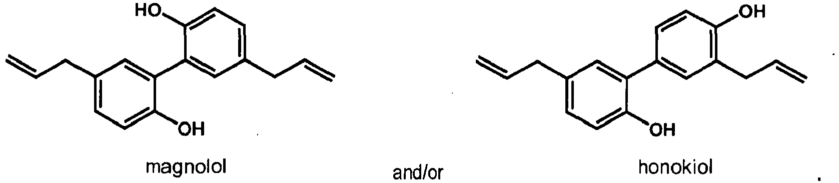 and
b) one or more oils having a polarity of 20 mN/m to 30 mN/m, **characterized in that** the oils having a polarity of 20 mN/m to 30 mN/m are selected from the group of compounds of ethylhexyl esters of fatty acids.

3. Cosmetic preparation according to either of the preceding claims, **characterized in that** the oils having a polarity of 20 mN/m to 30 mN/m are selected from the group of compounds comprising ethylhexyl cocoate (INCI Ethylhexyl Cocoate) and ethylhexyl stearate (INCI Ethylhexyl Stearate).

4. Cosmetic preparation according to any of the preceding claims, **characterized in that** the preparation comprises oils having a polarity of up to in a total concentration of 0.5 to 5% by weight, based on the total weight of the preparation.

5. Cosmetic preparation according to any of the preceding claims, **characterized in that** the preparation is in the form of an O/W emulsion.

6. Cosmetic preparation according to any of the preceding claims, **characterized in that** the preparation comprises sodium stearoyl glutamate.

7. Cosmetic preparation according to any of the preceding claims, **characterized in that** the preparation comprises sodium stearoyl glutamate at a concentration of 0.1 to 0.3% by weight, based on the total weight of the preparation.

8. Cosmetic preparation according to any of the preceding claims, **characterized in that** the preparation comprises cyclomethicone.

9. Cosmetic preparation according to any of the preceding claims, **characterized in that** the preparation comprises cyclomethicone at a concentration of 4 to 18% by weight, based on the total weight of the preparation.

10. Cosmetic preparation according to any of the preceding claims, **characterized in that** the preparation comprises magnolia officinalis bark extract at a concentration of 0.01 to 0.5% by weight, based on the total weight of the preparation.

## Revendications

1. Préparation cosmétique contenant :
a) Magnolia Officinalis Bark Extract et
b) une ou plusieurs huiles ayant une polarité de 20 mN/m à 30 mN/m, **caractérisée en ce que** les huiles ayant une polarité de 20 mN/m à 30 mN/m sont choisies dans le groupe de composés constitué par les esters éthylhexyliques d'acides gras.

2. Préparation cosmétique contenant :
a) 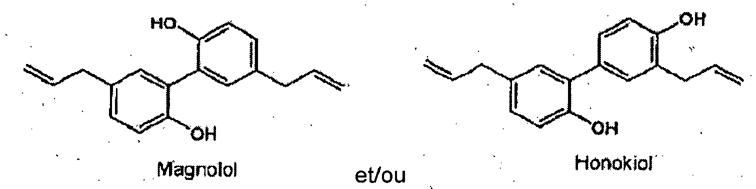 ainsi que
b) une ou plusieurs huiles ayant une polarité de 20 mN/m à 30 mN/m, **caractérisée en ce que** les huiles ayant une polarité de 20 mN/m à 30 mN/m sont choisies dans le groupe de composés constitué par les esters éthylhexyliques d'acides gras.

3. Préparation cosmétique selon l'une quelconque des revendications précédentes, **caractérisée en ce que** les huiles ayant une polarité de 20 mN/m à 30 mN/m sont choisies dans le groupe de composés constitué par le cocoate d'éthylhexyle (INCI Ethylhexyl Cocoate) et le stéarate d'éthylhexyle (INCI Ethylhexyl Stearate).

4. Préparation cosmétique selon l'une quelconque des revendications précédentes, **caractérisée en ce que** la préparation contient des huiles ayant une polarité de jusqu'à en une concentration totale de 0,5 à 5 % en poids, par rapport au poids total de la préparation.

5. Préparation cosmétique selon l'une quelconque des revendications précédentes, **caractérisée en ce que** la préparation se présente sous la forme d'une émulsion H/E.

6. Préparation cosmétique selon l'une quelconque des revendications précédentes, **caractérisée en ce que** la préparation contient du glutamate de stéaroyle sodique.

7. Préparation cosmétique selon l'une quelconque des revendications précédentes, **caractérisée en ce que** la préparation contient du glutamate de stéaroyle sodique en une concentration de 0,1 à 0,3 % en poids, par rapport au poids total de la préparation.

8. Préparation cosmétique selon l'une quelconque des revendications précédentes, **caractérisée en ce que** la préparation contient de la cyclométhicone.

9. Préparation cosmétique selon l'une quelconque des revendications précédentes, **caractérisée en ce que** la préparation contient de la cyclométhicone en une concentration de 4 à 18 % en poids, par rapport au poids total de la préparation.

10. Préparation cosmétique selon l'une quelconque des revendications précédentes, **caractérisée en ce que** la préparation contient Magnolia Officinalis Bark Extract en une concentration de 0,01 à 0,5 % en poids, par rapport au poids total de la préparation.
